# EUROPEAN PATENT APPLICATION

(11) **EP 4 335 919 A1**
(43) Date of publication of application: **13.03.2024**
(21) Application number: 22799115.5
(22) Date of filing: 04.05.2022
(51) Int. Cl.: C12N 5/0784, C07K 14/78, A61K 35/15, A61P 35/00, A61P 31/00, A61P 29/00, A61P 37/00

(54) **METHOD FOR CULTURING DENDRITIC CELLS**

(30) Priority: 07.05.2021 KR 20210059019; 28.04.2022 KR 20220052836
(71) Applicant: Nanofaentech Co., Ltd., Gimhae-si, Gyeongsangnam-do 50969 (KR)
(72) Inventor: KWAK, Jong Young, Suwon-si, Gyeonggi-do 16496 (KR); MAZA, Perry Ayn Mayson, Cabanatuan City, 3100 (PH)
(74) Representative: dompatent von Kreisler Selting Werner - Partnerschaft von Patent- und Rechtsanwälten mbB
(86) International application number: PCT/KR2022/006397
(87) International publication number: WO 2022/235072

(57) **Abstract**

The present invention relates to a composition for inhibiting self-activation of primary dendritic cells derived from lymphoid tissue isolated from the spleen, including, as active ingredients, PHSRN, RGD and LDV peptides derived from the integrin-binding domain of fibronectin and peptides including these amino acids, and relates to the efficacy of a peptide combination for inhibiting self-activation, presenting a culture method in which, when primary dendritic cells isolated from lymphoid tissue are cultured, the self-activity thereof is inhibited by the addition of fibronectin protein, but there is a disadvantage in that the activation thereof is not caused by an immunostimulatory substance, whereas the activation of the primary dendritic cells cultured by adding the peptides can be normally induced by an immunostimulant. The primary lymphoid tissue dendritic cells activated while being cultured with the peptide combination enhance the proliferation of T cells and induce the production of cytokines such as interferon upon co-culture with T cells. The compound can be effectively used as a composition for the prevention or treatment of cancer, autoimmune diseases and inflammatory diseases, through dendritic cell culture, the induction of dendritic cell activation, and T cell proliferation by co-culture with dendritic cells.

## Description

### [Technical Field]

The present invention relates to a method for culturing immature primary dendritic cells isolated from lymphoid tissue, and more specifically, to a culturing method capable of culturing dendritic cells while maintaining a state that does not cause activation due to a combination of fibronectin-derived peptides and linker peptides composed of these peptides.

### [Background Art]

Dendritic cells (DCs) are antigen presenting cells that provide association between innate and adaptive immune responses. The dendritic cells are widely distributed throughout the body and are divided into several subtypes in the spleen, lymph nodes, mucous membranes, intestine and epidermal tissues. In the spleen, dendritic cell subtypes are well distinguished from macrophage and monocyte types and characterized.

Inactive dendritic cells express major histocompatibility class-II (MHC-II) surface molecules and co-stimulatory molecules at a low level, and produce almost no inflammation-inducing cytokines. Dendritic cells can directly detect pathogens by pattern recognition receptors such as scavenger receptors, toll-like receptors, mannose receptors, etc., and the noxious signals derived from pathogens or damaged cells and antigens around the same are subjected to phagocytosis through a phagocytosis mechanism. The activated dendritic cells are converted to a cell type with reduced antigen uptake capacity, increased expression of MHC-II, increased expression of costimulatory molecules such as CD80, CD86 and CD40, and a chemoreceptor such as CCR7 by these receptors, and are characterized by the secretion of different types of cytokines and increased migration to the lymph nodes. These activated dendritic cells strongly induce T-cell responses to specific antigens.

Antigen phagocytosis and processing are accomplished by the inactive dendritic cells. The biggest problem in measuring the immunological function of these stable dendritic cells and using the same as a cell therapy agent is the isolation and culture of tissue-derived primary dendritic cells (DCs). DCs isolated from lymphoid tissues for cell culture undergo spontaneous activation from an inactive form to an active form within 12 hours in a culture state. Since naturally activated dendritic cells have a very short survival period, long-term culture is impossible. Self activation was observed to be slightly decreased when the number of dendritic cells was reduced in culture conditions or cultured on a semi-solid gel, etc., therefore, it was proposed that such self activation occurs by a combination of dendritic cells or contact therebetween. However, specific factors associated with the activation or a technology capable of cultivating the dendritic cells while maintaining the inactive form thereof have yet been reported.

A three-dimensional structure of the spleen and lymph nodes and an extracellular matrix regulate an interaction between cells and play an important role in the aggregation of cells in tissues. Only a very small amount of collagen is present in the paracortical region of lymph nodes, and fibronectin is expressed as a major extracellular matrix in lymphoid tissues such as the spleen, while laminin expression is extremely low. It has been reported that dendritic cells differentiated from monocytes using cytokines change their shape and maintain immature form when culturing them on a plate coated with fibronectin or laminin.

Because of such various technical limitations as described above, rather than culturing primary dendritic cells isolated from lymphoid tissue, a technology using a transformed dendritic cell line by genome transformation or dendritic cells differentiated from precursor cells is applied. Recently, as the most common method, the dendritic cells used for culture are dendritic cells differentiated from bone marrow cells or monocytes, which are cells differentiated from precursor cells in the presence of inflammatory factors such as granulocyte macrophage-colony stimulating factor (GM-CSF) and tumor necrosis factor (TNF-α). Therefore, the dendritic cells differentiated from these precursor cells can be induced by cell culture of bone marrow cells or monocytes, but belong to inflammatory dendritic cells wherein monocytes escape *in vivo* from the blood to the tissue in an inflammatory state and appear *in vivo.* However, in this case, dendritic cells activated in the process of differentiation from monocytes rather than primary dendritic cells present in lymph nodes can maintain relatively stable functions in the culture state, unlike unstimulated dendritic cells. Further, a culture method that can stably cultivate dendritic cells isolated from the spleen in an inactive state using a cell culture structure based on nano-micro hybrid fibers, as well as a method for analysis of inactive dendritic cells, have been reported.

In the present invention, there is provided a technology in relation to dendritic cells, wherein the cells exist in a functionally inactive state to phagocyte antigens while maintaining the survival of primary dendritic cells isolated from immune tissues, and are modified into an active form when subjected to immune or inflammatory stimulation, whereby immunological responses of dendritic cells possibly occurring *in vivo* can be implemented in the culture state. The isolated primary dendritic cells induce the proliferation of T cells, or are co-cultured with T cells to induce cytokine secretion. Therefore, these dendritic cells have high possibility for development of an immune cell therapeutic agent for diseases such as cancer, viral infection, autoimmune disease, organ transplantation, allergy, and rheumatoid arthritis.

### [Summary of Invention]

### [Problems to be Solved by Invention]

An object of the present invention is to provide a composition for inhibiting self-activation of dendritic cells, which includes a specific peptide capable of maintaining primary dendritic cells isolated from lymphoid tissues in an inactive form.

In addition, another object of the present invention is to provide a method for activating immune cells, which includes co-culturing dendritic cells and immune cells cultured by the above culture method.

Further, another object of the present invention is to provide a dendritic cell composition, which includes isolated dendritic cells maintained in an inactive state for 24 hours or more, as well as a pharmaceutical composition for treating or preventing cancer, which includes the above composition as an active ingredient.

### [Means for Solving Problems]

1. A composition for inhibiting self-activation of dendritic cells, including a fibronectin-derived peptide which binds to an integrin binding site between the dendritic cells.
2. The composition according to the above 1, wherein the peptide is a peptide consisting of an amino acid sequence including any one of SEQ ID NOs: 1 to 3.
3. The composition according to the above 1, wherein the peptide has any one amino acid sequence of SEQ ID NOs: 1 to 3, and has a length of 3 to 11aa.
4. The composition according to the above 1, wherein the peptide is at least one selected from the group consisting of any one amino acid sequence of SEQ ID NOs: 1 to 7.
5. The composition according to the above 1, wherein the peptide is a peptide in which two or more amino acid sequences including any one of SEQ ID NOs: 1 to 3 are linked by a linker.
6. The composition according to the above 5, wherein the linker consists of amino acids G and S, and has a length of 2 to 8aa.
7. The composition according to the above 5, wherein the linker consists of an amino acid sequence including any one of SEQ ID NOs: 8 to 11.
8. The composition according to the above 5, wherein the peptide has any one amino acid sequence of SEQ ID NOs: 12 to 14.
9. The composition according to the above 1, wherein the peptide is contained in a concentration of 200 ug/mL or more based on 2 × 10 ⁵/mL of the dendritic cells to be treated.
10. The composition according to the above 1, wherein the dendritic cells are derived from monocytes, spleen or lymph nodes.
11. The composition according to the above 1, wherein the composition is for use *in vitro.*
12. A method of culturing dendritic cells, including treating the isolated dendritic cells with the composition according to any one of the above 1 to 11.
13. The method according to the above 12, further including inducing activation of the dendritic cells by treating the dendritic cells, which were treated with the composition, with an immunostimulant.
14. The method according to the above 13, wherein the immunostimulant is at least one selected from the group consisting of a bacterial agent, CD40 ligand, TNF-α, IL-1β, LPS and poly (I:C).
15. A method of activating immune cells, including: treating the isolated dendritic cells with the composition according to any one of the above 1 to 11 and culturing the dendritic cells in an inactivated state;
   activating the dendritic cells; and
   co-culturing the activated dendritic cells and immune cells.
16. A dendritic cell composition, including: a fibronectin-derived peptide which binds to an integrin binding site between the dendritic cells according to any one of the above 1 to 11; and dendritic cells mixed therewith.
17. A pharmaceutical composition for treating or preventing cancer, infectious disease, chronic inflammatory disease, or autoimmune disease, including the cell composition according to the above 16 as an active ingredient.

### [Advantageous effects]

The present invention relates to a cell culture composition for maintaining dendritic cell inactivity, which includes, as an active ingredient, a peptide combination containing a cell adhesive domain sequence among fibronectin protein sequences, and relates to efficacy of the dendritic cells derived from the spleen and lymph tissues such as lymph nodes, as a culturing agent. The peptide combination may maintain the inactivation of dendritic cells or activate cells by immunostimulants, and induce proliferation and activation of T cells, whereby the peptide combination may be effectively used as a composition for immune-diagnosis of cancer, autoimmune disease and inflammatory disease or for immune cell therapy.

### [Brief Description of Drawings]

FIG. 1 shows results of observing the distribution of spleen dendritic cells (spleen DC, sDC) cultured without adding a peptide mixture of the present invention through a confocal microscope, and the degree of cell clustering.
FIG. 2 shows flow cytometric analysis results of spleen dendritic cells cultured without adding the peptide mixture of the present invention and a stimulant, which are results of confirming that the expression of MHC-II, CD86, CD80 and CD40 is increased in a time-dependent manner on the surface of the dendritic cells.
FIG. 3 shows results of confirming the distribution of spleen dendritic cells cultured after adding fibronectin or laminin without adding the peptide mixture of the present invention through a confocal microscope.
FIG. 4 shows flow cytometric analysis results of measuring the expression levels of CD86 and MHC-II on the surface of spleen dendritic cells after adding fibronectin or laminin at different concentrations without adding the peptide mixture of the present invention and then culturing for 6 hours.
FIG. 5 shows flow cytometric analysis results of measuring the expression levels of CD86, MHC-II, CD80 and CD40 on the cell surface of spleen dendritic cells after adding fibronectin or laminin by concentration of 200 ug/mL without adding the peptide mixture of the present invention and then culturing for 6 hours.
FIG. 6 shows the amino acid sequence and peptide types of the peptide used in the present invention.
FIG. 7 shows flow cytometric analysis results of measuring the expression level of CD86 after culturing spleen dendritic cells for 6 hours with addition of the peptide mixture of the present invention.
FIG. 8 shows results of observing the distribution of spleen dendritic cells after culturing for 6 hours or 24 hours with addition of the peptide mixture of the present invention through a confocal microscope.
FIG. 9 shows flow cytometric analysis results of measuring the expression level of CD86 after culturing spleen dendritic cells for 6 hours with addition of a peptide mixture containing P3.
FIG. 10 shows flow cytometric analysis results of measuring the expression levels of MHC-II, CD86, CD80 and CD40 in spleen dendritic cells after culturing for 6 hours or 24 hours with addition of three peptide mixtures in various combinations, as compared to the culture state of adding fibronectin.
FIG. 11 shows results of observing CD86 expression after culturing dendritic cells with addition of the peptide mixture of the present invention, and then fluorescence staining for CD86 expression with an antibody, through a confocal microscope.
FIG. 12 shows the enzyme-linked immunosorbent assay (ELISA) results of measuring the amount of TNF-α secreted in a culture medium of dendritic cells cultured without addition of the peptide mixture of the present invention over time.
FIG. 13 shows ELISA results of measuring the amount of secreted TNF-α after culturing for 24 hours with addition of three peptide mixtures.
FIG. 14 shows flow cytometric analysis results of measuring the expression level of CD86 in spleen dendritic cells, which were cultured for 24 hours with or without addition of lipopolysaccharide (LPS) as an immunostimulant to a culture plate containing three mixtures or fibronectin added thereto.
FIG. 15 shows flow cytometric analysis results of measuring the expression level of CD86 in spleen dendritic cells, which were cultured for 24 hours with or without addition of LPS as an immunostimulant to a polycaprolactone (PCL) nanofiber membrane containing a peptide mixture or fibronectin added thereto.
FIG. 16 shows results of measuring the amounts of TNF-α and IL-12p40 secreted from spleen dendritic cells by ELISA, wherein the dendritic cells were cultured for 24 hours with or without addition of LPS as an immunostimulant to a culture plate containing three peptide mixtures or fibronectin added thereto.
FIG. 17 shows flow cytometric analysis results of measuring the expression level of CD86 in bone marrow-derived DC (BMDCs), which were cultured for 24 hours with or without addition of LPS as an immunostimulant to a culture plate containing three peptide mixtures or fibronectin added thereto.
FIG. 18 shows flow cytometric analysis results of measuring the expression level of CD86 in BMDCs, which were cultured for 24 hours with addition of LPS to a culture plate containing three peptide mixtures or fibronectin at different concentrations added thereto.
FIG. 19 shows ELISA results of measuring the amounts of TNF-α and IL-12p40 secreted from BMDCs, which were cultured for 24 hours with addition of LPS to a culture plate containing three peptide mixtures or fibronectin at different concentrations added thereto.
FIG. 20 shows flow cytometric analysis results of measuring the expression level of CD86 in spleen dendritic cells compared to LPS, wherein the dendritic cells were obtained by adding poly (I:C) as an immunostimulant to a culture plate containing three peptide mixtures or fibronectin added thereto and culturing the same for 24 hours.
FIG. 21 shows flow cytometric analysis results of T cell proliferation by mixed lymphocyte reaction in a mixture of spleen dendritic cells and T cells isolated from allogeneic mice and fluorescence-stained with carboxyfluorescein succinimidyl ester (CFSE), wherein the dendritic cells were obtained by adding LPS or poly (I:C) as an immunostimulant to a culture plate containing three peptide mixtures or fibronectin added thereto and culturing the same for 24 hours.
FIG. 22 shows results of measuring the amounts of cytokines secreted after 4 days co-culture of spleen dendritic cells and T cells isolated from allogeneic mice by ELISA, wherein the spleen dendritic cells were obtained by adding LPS or poly (I:C) as an immunostimulant to a culture plate containing three peptide mixtures or fibronectin added thereto and then culturing the same for 24 hours.
FIG. 23 shows results of measuring the viability of spleen primary dendritic cells by flow cytometry through 7-aminoactinomycin D (7-AAD) staining, wherein the dendritic cells were cultured for 6 hours in a culture plate containing three peptide mixtures or fibronectin added thereto.
FIG. 24 shows results of measuring the expression level of CD86 in the spleen dendritic cells by flow cytometry, wherein the dendritic cells were cultured after adding the linker peptide of the present invention thereto.
FIG. 25 shows flow cytometric analysis results of measuring the expression level of CD86 in spleen primary dendritic cells cultured by adding the scrambled linker peptide of the present invention thereto.
FIG. 26 shows results of confirming the expression of CD86 after adding a linker peptide to spleen dendritic cells and culturing the same for 24 hours through a confocal microscope.
FIG. 27 shows ELISA results of measuring the concentration of tumor necrosis factor (TNF-α) secreted after adding a linker peptide or LPS to spleen primary dendritic cells and culturing the same for 24 hours.
FIG. 28 shows flow cytometric analysis results of measuring T cell proliferation after co-culture of spleen dendritic cells, in which self-activation does not occur after culturing with a linker peptide, or dendritic cells activated with LPS, as well as OT-1 T cells which were fluorescence-stained with CFSE, for 4 days.
FIG. 29 shows ELISA results of measuring the concentrations of interferon-gamma (IFN-γ) and TNF-α secreted in the culture medium after co-culture of spleen dendritic cells, which were cultured with a linker peptide, and OT-1 T cells, for 4 days.

### [Mode for Carrying out Invention]

The present invention provides a composition for inhibiting self-activation of dendritic cells *in vitro,* which includes a fibronectin-derived peptide that binds to an integrin binding site between the dendritic cells.

Dendritic cells treat pathogenic substances using immune cells and display antigens on the surface for other cells of the immune system. That is, the dendritic cells play a role of antigen presenting cells, therefore, can function as a mediator between innate immune response and acquired immune response. The type of dendritic cells may be, for example, monocyte-derived dendritic cells, spleen-derived dendritic cells, or lymphoid tissue-derived dendritic cells, but it is not limited thereto.

The integrin binding site between the dendritic cells may be a site playing an important role in cell-matrix and cell-cell binding and interaction, and when the peptide binds to such an integrin binding site, cell-cell interaction is inhibited. Therefore, cell-to-cell signaling and self-activation can be inhibited.

The fibronectin-derived peptide may consist of a part of the sequence of fibronectin, which is one of the extracellular matrix proteins, and may additionally include an amino acid sequence other than the part of the sequence of fibronectin.

That is, the present invention may include a composition for inhibiting self-activation of dendritic cells, which includes a peptide having an amino acid sequence at a site bound to an integrin binding site between the dendritic cells in fibronectin.

The fibronectin sequence may specifically include a cell adhesion domain sequence among domains constituting fibronectin.

The peptide may consist of a sequence including RGD (SEQ ID NO: 1), LDV (SEQ ID NO: 2) or PHSRN (SEQ ID NO: 3), for example, RGD (SEQ ID NO: 1), LDV (SEQ ID NO: 2), PHSRN (SEQ ID NO: 3), KGRGDS (SEQ ID NO: 4), GRGDS (SEQ ID NO: 5), GGPEILDVPST (SEQ ID NO: 6) or EILDVPST (SEQ ID NO: 7).

The peptide may have a length of 3 to 11aa, but it is not limited thereto.

The peptide may be a peptide in which two or more amino acid sequences including RGD, LDV or PHSRN are linked by a linker.

The linker is composed of amino acids G and S, and may have a length of 2 to 8aa (amino acid). For example, the linker may be one consisting of an amino acid sequence including GS (SEQ ID NO: 8), GGGS (SEQ ID NO: 9), GGGGS (SEQ ID NO: 10) or GGGSGGGS (SEQ ID NO: 11).

For example, the peptide may include the amino acid sequence of PHSRNGSRGDGSLDV (SEQ ID NO: 12), PHSRNGGGSRGDGGGSLDV (SEQ ID NO: 13) or PHSRNGGGSGGGSRGDGGGSLDV (SEQ ID NO: 14).

In the present embodiment, the 'linker peptide' refers to a peptide in which a plurality of peptides are linked by a linker as described above.

The composition may include two or more types of the peptide.

In order to inhibit the self-activation of dendritic cells, the composition of the present invention may contain the peptide at a specific concentration or more based on 2 × 10⁵/mL dendritic cells, and the concentration may be, for example, 50 ug/mL or more, 100 ug/mL or more, 200 ug/mL or more, 300 ug/mL or more, 400 ug/mL or more, or 500 ug/mL or more, specifically 50 to 800 µg/mL, 50 to 500 ug/mL, 100 to 800 ug/mL, 100 to 600 ug/mL, 100 to 500 ug/mL, 100 to 300 ug/mL, 100 to 200 µg/mL, 150 to 250 ug/mL, 200 to 800 ug/mL, 200 to 500 ug/mL or 300 to 500 ug/mL, but it is not limited thereto.

The composition of the present invention can modulate a dendritic cell-mediated immune response, specifically, it can inhibit the self-activation of dendritic cells or regulate the self-antigen presenting ability of dendritic cells, but it is not limited thereto.

Self-activation of dendritic cells refers to a spontaneous activation phenomenon in which isolated dendritic cells are converted from an inactive form to an active form within 12 hours in the culture state. It has been reported that the above-described phenomenon occurs due to the binding or contact between the dendritic cells. Since the activated dendritic cells have a very short survival period and a disadvantage that these cannot be cultured for a long period of time, to inhibit this problem makes it possible to culture the isolated dendritic cells in an inactive form that enables phagocytosis and processing of antigens.

The composition for inhibiting activation according to the present invention may be used *in vivo* or *in vitro,* but preferably for use *in vitro.*

Further, the present invention provides a method for culturing dendritic cells, which includes treating the composition for inhibiting the self-activation of dendritic cells.

The composition for inhibiting self-activation is the same as described above, and a treatment concentration and a treatment time of the composition are not limited to specific concentration and time as long as it can inhibit the self-activation of dendritic cells. The treatment concentration may be, for example, 50 to 500 ug/mL, 100 to 400 ug/mL or 0 to 300 ug/mL per cell number 2 × 10⁵/mL, and the treatment time may be, for example, 2 to 48 hours, 6 to 48 hours, 4 to 24 hours, 6 to 18 hours, 4 to 12 hours, 6 to 8 hours, or 6 to 24 hours, but it is not limited thereto.

The method for culturing dendritic cells of the present invention may further include inducing activation of dendritic cells by treatment with an immunostimulant, wherein the immunostimulant refers to a substance that exposes an antigen to the dendritic cells and causes the cells to be converted into cells displaying the antigen on the surface, and types thereof may include, for example, bacterial formulations, CD40 agonists, TNF-α, IL-1β, LPS and poly (I:C), but it is not limited thereto.

The dendritic cell culture may be cultured in three dimensions on a membrane composed of polycaprolactone (PCL) nanofibers, but it is not limited thereto.

Further, the present invention provides a method for activating immune cells.

The method for activating immune cells may include: treating the isolated dendritic cells with the composition described above and culturing the dendritic cells in an inactivated state; activating the dendritic cells; and co-culturing the activated dendritic cells and immune cells.

The immune cell is a cell involved in an immune response in the immune system, and may refer to a cell used as a cell therapeutic agent, and types thereof may include, for example, T cells, B cells, natural killer cells (NK cells), natural killer T cells (NKT cells), mast cells, or bone marrow-derived phagocytes, but they are not limited thereto.

The step of culturing the dendritic cells in an inactivated state by treatment of the composition is the same as described above in the dendritic cell culture method.

The step of activating the dendritic cells may be performed by treating the inactive form of dendritic cells with a maturation factor or an immunostimulant, wherein types of the maturation factor or the immunostimulant are not particularly limited as long as it is a substance to induce an increase in expression of MHC-II, CD86, CD80 or CD40 on the surface of the dendritic cells, and may include, for example, a bacterial agent, CD40 agonists, TNF-α, IL-1β, LPS or poly (I:C).

The step of co-culturing the activated dendritic cells and immune cells is for inducing proliferation of immune cells and secretion of cytokines. For example, a ratio of the number of co-cultured immune cells based on the activated dendritic cells may be 1 to 50, 4 to 30, 5 to 25, 10 to 30 or 15 to 25, and a co-culture time may be 2 to 144 hours, 4 to 120 hours, 12 to 96 hours, 24 to 96 hours, 48 to 96 hours, or 48 to 72 hours, but it is not limited thereto.

Further, the present invention also provides a dendritic cell composition, including the peptide and dendritic cells mixed therewith.

The dendritic cells of the composition may be maintained in an inactive state for, for example, 6 hours or more, 12 hours or more, 24 hours or more, or 48 hours or more, and preferably 6 to 24 hours, 6 to 48 hours, 12 to 48 hours, or 12 to 24 hours, but it is not limited thereto.

The cell composition may include the dendritic cells maintained in an inactive state by the above-described composition for inhibiting the activation of dendritic cells, wherein the dendritic cells may be dendritic cells specifically inhibited in terms of the increase in the expression of MHC-II, CD86, CD80 or CD40.

The dendritic cells may functionally conduct phagocytosis of antigens while maintaining survival until these are stimulated and activated, and unlike the activated dendritic cells, the dendritic cells may be cultured for a long period of time to induce proliferation of T cells *in vitro,* or may be co-cultured with T cells to accelerate cytokine secretion, whereby it is possible to develop immune cell therapeutics for various diseases.

The present invention also provides a pharmaceutical composition for treating or preventing cancer, bacterial and viral infectious diseases, chronic inflammatory diseases, or autoimmune diseases, which includes the dendritic cell composition as an active ingredient.

The cancer may be, for example, lung cancer, laryngeal cancer, stomach cancer, colon/rectal cancer, liver cancer, gallbladder cancer, pancreatic cancer, breast cancer, ovarian cancer, uterine cancer, cervical cancer, prostate cancer, kidney cancer, epithelial cell-derived carcinoma, bone cancer, muscle cancer, adipose cancer, connective tissue cell-derived sarcoma, leukemia, lymphoma, hematological cancer derived from hematopoietic cells of multiple myeloma, tumor arising from neural tissue, thyroid cancer, colon cancer, testicular cancer, brain tumor, head and neck cancer, bladder cancer, epithelial cancer, adenocarcinoma, esophageal cancer, mesothelioma, skin cancer or melanoma, but it is not limited thereto.

The infectious disease may be a disease caused by infection with a pathogen such as bacteria, spirochete, rickettsia, viruses, fungi, or parasites, but it is not limited thereto.

The chronic inflammatory disease may be a disease caused by the accumulation of inflammation for a long time, and is known to disturb the immune system by causing cellular aging and transformation and excessively activating the immune response. For these reasons, it has been reported that this disease induces even metabolic diseases such as obesity, diabetes, etc. or autoimmune disease.

The autoimmune disease is a disease occurring when the internal immune system attacks internal normal cells rather than external antigens, and may include, for example, rheumatoid arthritis, atopy, lupus, Crohn's disease, type 1 diabetes, ulcerative colitis, multiple sclerosis, and the like, but it is not limited thereto.

Other than the cancer, infectious disease or immune disease described above, for any disease in which immune cells can be used as a therapeutic agent, regardless of types thereof, a pharmaceutical composition for treating or preventing the disease using the cell composition of the present invention as an active ingredient may be used.

Hereinafter, this invention will be described in detail by means of examples. The following examples are illustrative of the present invention. Therefore, the contents of this invention are not limited to the following examples.

### Example 1. Isolation and culture of spleen dendritic cells

Mouse spleen dendritic cells were obtained by adding EDTA-BSS (Balanced Salt Solution) buffer and collagenase solution (DMEM containing 100 units of collagenase type IV, medium containing low glucose) to spleens of female C57BL/6 mice and cutting it with surgical scissors, mixing the same with 6 mL of collagenase treatment solution, and incubating the mixture for 30 minutes. The separated whole spleen tissue was filtered using a 70 um cell strainer (Falcon), followed by centrifugation at 1800 rpm for 5 minutes thus to separate the cells. Red blood cells were removed by adding red blood cell lysis buffer (Sigma-Aldrich) to the cells and washed twice with RPMI-1640 medium containing 10% fetal bovine serum (FBS). Dendritic cells were reacted with CD11c⁺ antibody-bound microbeads and isolated using CD11c Microbeads Ultrapure (MACS, Milteni Biotec, USA).

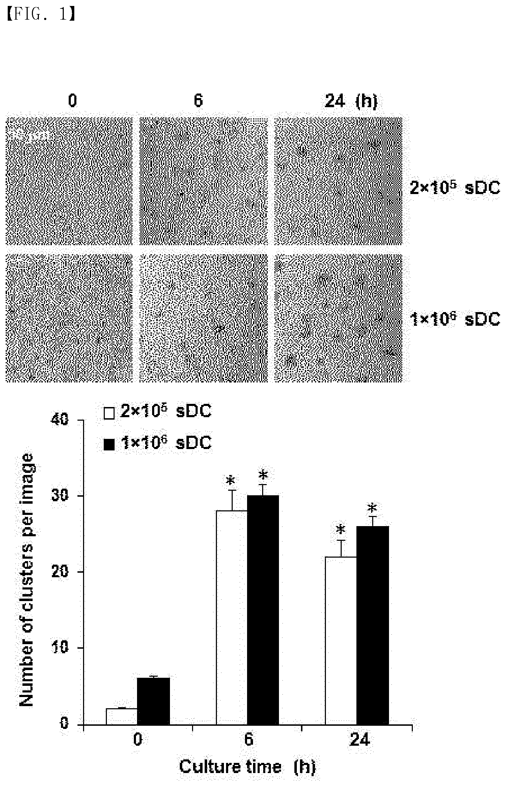

In FIG. 1, primary dendritic cells isolated from the spleen were dispensed in a 48-well plate and cultured for 24 hours in a cell chamber maintained at 37°C and 5% CO₂, and the extent of clustering in which cells were distributed or gathered into single cells was evaluated. When observed by differential interference contrast (DIC) imaging of a confocal microscope (Olympus confocal microscope FV1000, Olympus Corporation; n=5), it was found that the aggregation of cells was further increased with the passage of culture time. Such phenomenon appeared more significantly at the density of 1 × 10⁶/mL than 2 × 10⁵/mL. The number of single cells and intercellular clusters was measured using ImageJ software (NIH, Bethesda). * indicates statistical significance as P <0.05 or less compared with 0 h.

### Example 2. Measurement of activation of dendritic cells using flow cytometry

In the unstimulated normal state, dendritic cells have an inactive phenotype with very low expression of MHC-II and co-stimulatory molecules such as CD80, CD86 and CD40, however, in the activated dendritic cells, expression of these markers is increased significantly.

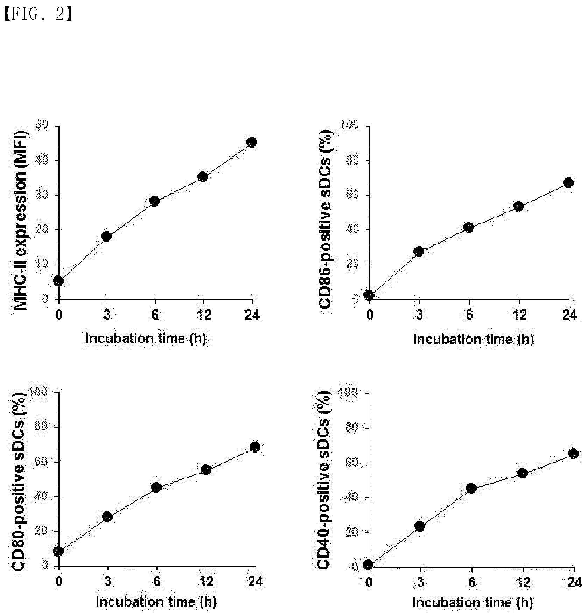

In FIG. 2, in order to determine whether self-activation occurs when primary dendritic cells isolated from the spleen are cultured, dendritic cells were dispensed in a 24-well plate at 2 × 10⁵/mL, and after culturing for 6 hours in RPMI-1640 medium at 37°C under 5% CO₂ condition, the expression levels of MHC-II, CD86, CD80 and CD40 were analyzed on the surface of dendritic cells using a flow cytometer (MACSQuant VYB flow cytometer, Miltenyi Biotec). Results measured using a flow cytometer were analyzed using FlowJo v10 (Tree Star, Ashland). The expression levels of CD86, CD80 and CD40 were shown as the percentage of cells stained with CD11c, and the expression level of MHC-II was shown as mean fluorescence intensity (MFI), which is the mean fluorescence intensity of fluorescence. As a result, the expression levels of MHC-II, CD86, CD80 and CD40 on the surface of dendritic cells were demonstrated to increase in a time-dependent manner.

### Example 3. Culture of spleen dendritic cells in a medium containing fibronectin added thereto

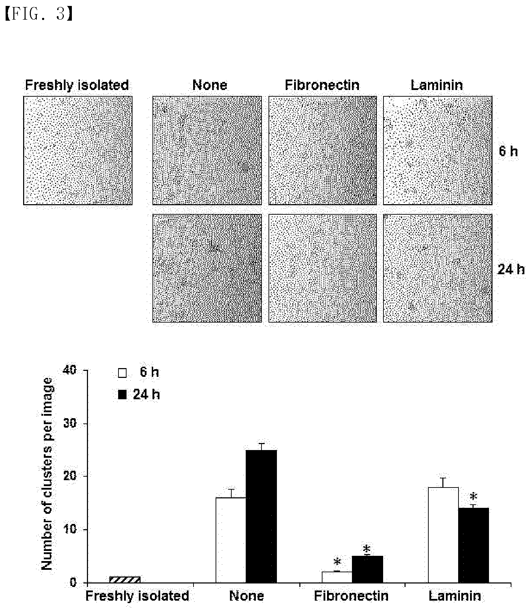

In FIG. 3, CD11c⁺ primary dendritic cells isolated from the spleen were dispensed at 2 × 10⁵/mL in RPMI-1640 medium after adding fibronectin or laminin at a concentration of 200 ug/mL thereto, and culture for 6 hours, followed by observing the distribution of cells using a confocal microscope. When fibronectin was added, cell aggregation was significantly reduced, suggesting the possibility that fibronectin is an extracellular matrix factor inhibiting aggregation between dendritic cells. Asterix (*) indicates statistical significance as P <0.05 or less compared with None.

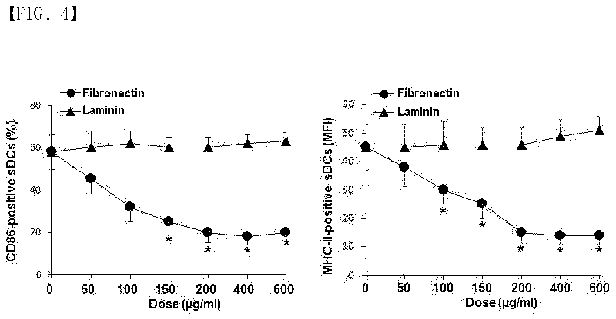

In FIG. 4, when fibronectin or laminin at various concentrations was added and spleen dendritic cells were cultured for 6 hours, followed by measuring the expression level of CD86 or MHC-II through flow cytometry, self-activation did not occur by fibronectin at a concentration of 200 ug/mL, and self-activation was not inhibited by laminin at a concentration of 200 ug/mL or higher. Therefore, it was confirmed in the present invention that fibronectin at a concentration of 200 µg/mL was the most effective for self-activation of spleen dendritic cells. * indicates statistical significance as P <0.05 or less compared to 0 ug/mL concentration.

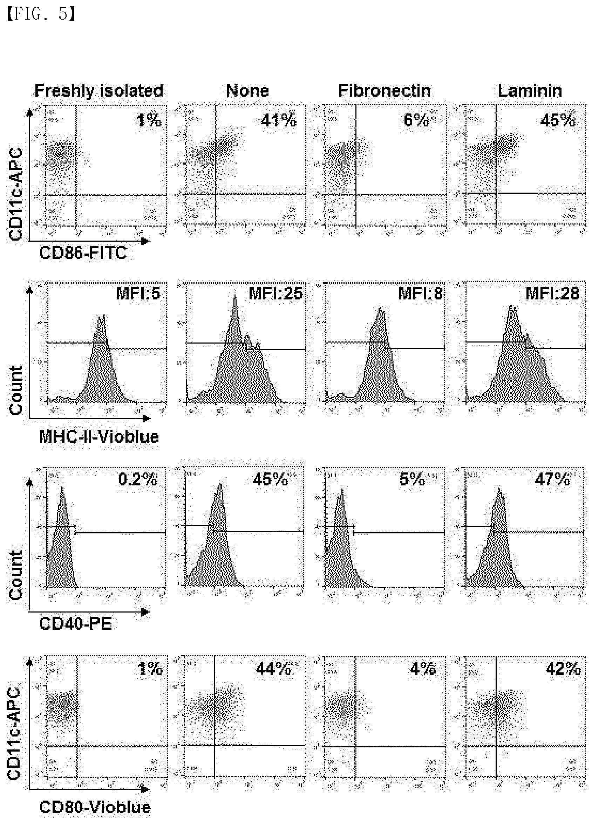

In FIG. 5, when CD11c⁺ spleen dendritic cells were cultured for 6 hours with fibronectin at a concentration of 200 ug/mL, it was confirmed that the expression of CD86 and MHC-II as well as the expression of CD80 and CD40 are not increased.

### Example 4. Culture of spleen dendritic cells in a medium added with a peptide prepared from the amino acid sequence of fibronectin protein

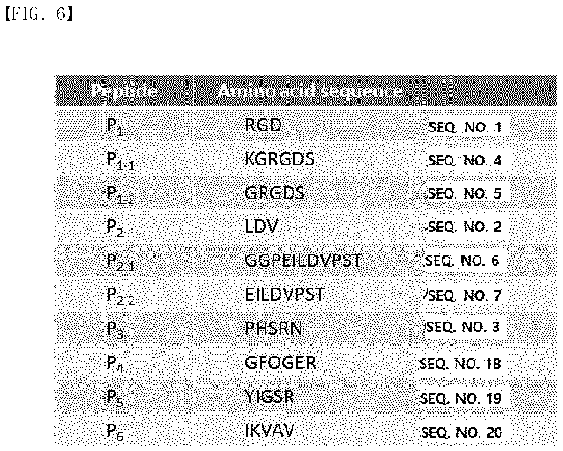

In FIG. 6, as an amino acid sequence in fibronectin, which is one of the extracellular matrix proteins mediating cell adhesion, Arginine-Glycine-Aspartate (Arg-Gly-Asp, RGD) (hereinafter referred to as P1, SEQ ID NO: 1) and the like corresponds to the site binding to the integrin protein of a cell membrane. In addition, the other amino acid sequences such as Leu-Asp-Val (LDV, SEQ ID NO: 2) (hereinafter referred to as P2) and Pro-His-Ser-Arg-Asn (PHSRN) (hereinafter referred to as P3), SEQ ID NO: 3) are also known to play the same role. Therefore, a peptide composed of these nucleotide sequences was requested to Peptron Co., Ltd. (Daejeon, Korea) and produced. Motifs such as RGD in the fibronectin protein bind to integrins and act on cell-matrix and cell-cell bonding. As similar peptides other than the RGD peptide, Lys-Gly-Arg-Gly-Asp-Ser (KGRGDS) (hereinafter referred to as P1-1, SEQ ID NO: 4), GRGDS (hereinafter referred to as P1-2, SEQ ID NO: 5), Gly-Gly-Pro-Glu-Ile-Leu-Asp-Val-Pro-Ser-Thr (GGPEILDVPST) (hereinafter referred to as P2-1, SEQ ID NO: 6), Glu-Ile-Leu-Asp-Val-Pro-Ser-Thr (EILDVPST) (hereinafter referred to as P2-2, SEQ ID NO: 7) were prepared and used. Further, Gly-Phe-Hyp-Gly-Glu-Arg (GFOGER) (hereinafter referred to as P4, SEQ ID NO: 18) as an amino acid sequence corresponding to the integrin binding motif in collagen, and Tyr-Ile-Gly-Ser-Arg (YIGSR) (hereinafter referred to as P5, SEQ ID NO: 19) and Ile-Lys-Val-Ala-Val (IKVAV) (hereinafter referred to as P6, SEQ ID NO: 20) as an amino acid sequence that is one of the integrin binding motifs in laminin, were prepared and used as controls.

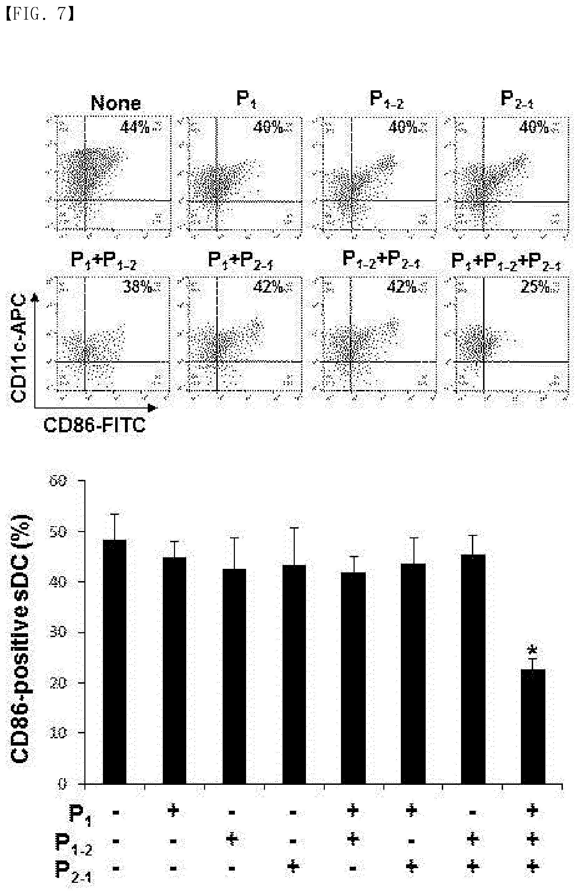

In FIG. 7, when culturing spleen dendritic cells, each of the prepared peptides was added at a concentration of 200 µg/mL and the expression level of CD86 was measured 6 hours after culture. When each peptide was used alone for treatment, the expression of CD86 was not inhibited in spleen dendritic cells. However, when the P1, P1-2, and P2-1 peptides were used together for treatment, the expression of CD86 was significantly reduced. Compared to when the cells were treated with these three kinds of peptides, the expression was not inhibited even when the two kinds of peptides were used together for treatment. * indicates statistical significance as P <0.05 or less compared to the P1, P1-2, and P2-1 conditions.

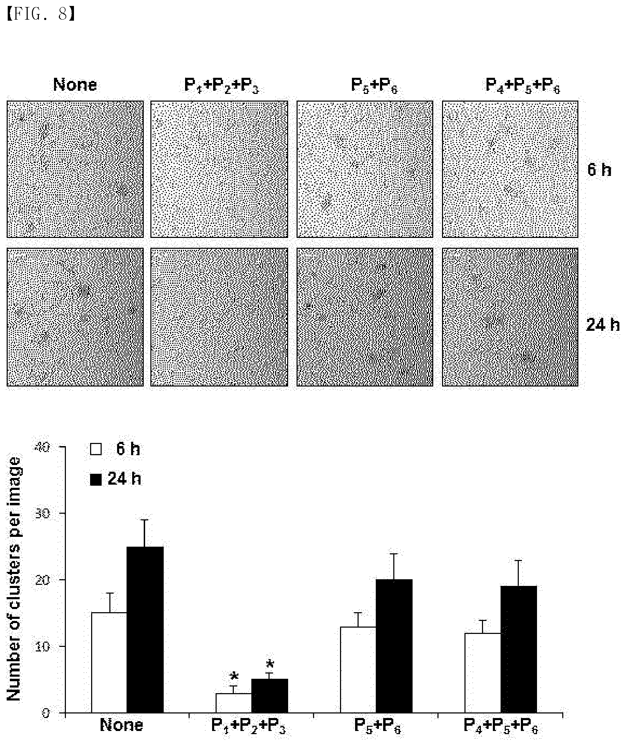

In FIG. 8, when the P3 peptide prepared was added in the spleen dendritic cell culture medium at a concentration of 200 µg/mL in combination with P1 (200 ug/mL) and P2 (200 ug/mL) and cultured for 6 hours or 24 hours, cell clustering was significantly inhibited. In comparison, it was found that cell aggregation was not inhibited even when three peptides, P4, P5 and P6, which are peptides derived from collagen or laminin protein, were added at the same time. Further, even when P5 and P6 as laminin-derived peptides were added, inhibition did not appear. * indicates statistical significance as P <0.05 or less compared with None.

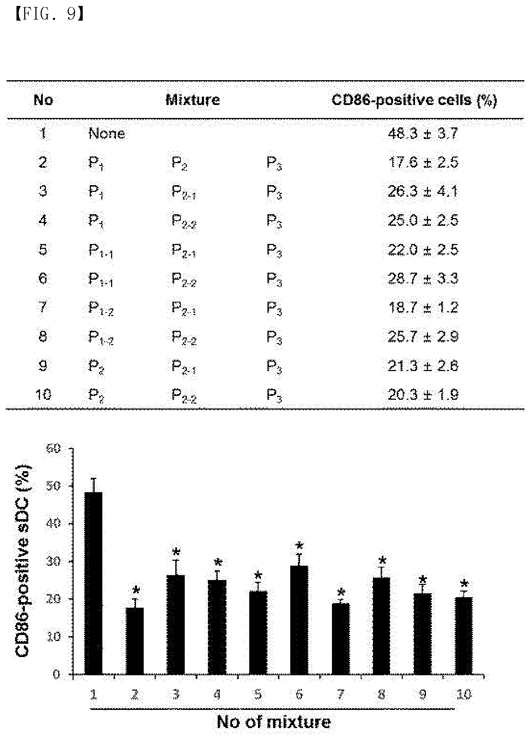

In FIG. 9, peptides containing P1 and P2 and amino acids of these peptides as well as P3 peptide in a combination of three types were added to the spleen dendritic cell culture medium, and after culturing for 6 hours, the expression level of CD86 was measured by flow cytometry. When the other two peptides containing P3 (200 ug/mL) were added at a concentration of 200 ug/mL, respectively, the expression of CD86 was significantly reduced in all measurements. Specifically, when P1, P2 and P3 peptides were added or P1-2, P2-1 and P3 peptides were added, the most significant inhibitory effect was exhibited. In comparison, when P3 was treated alone at a concentration of 600 µg/mL, no inhibitory effect was exhibited. * indicates statistical significance as P <0.05 or less compared with None.

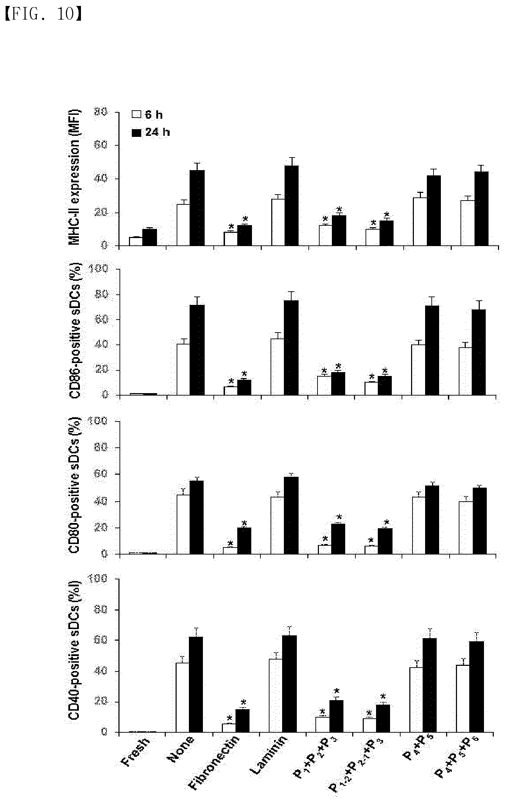

In FIG. 10, in the case where three types of peptides were added to the spleen dendritic cell culture medium and cultured for 6 hours or 24 hours, when it was measured whether to inhibit the expression of MHC-II, CD80 and CD40 in addition to CD86 by flow cytometry, it was confirmed that, if P1, P2 and P3 peptides are added as well as fibronectin or if P1-2, P2-1 and P3 peptides are added, the expression thereof is significantly inhibited. In comparison, when P4, P5 and P6 peptides were added at a concentration of 200 ug/mL, respectively, no inhibitory effect was observed. Further, even when laminin was treated alone, no effect was observed. * indicates statistical significance as P <0.05 or less compared with None.

### Example 5. Measurement of fluorescence staining of CD86 in spleen dendritic cells cultured with peptides

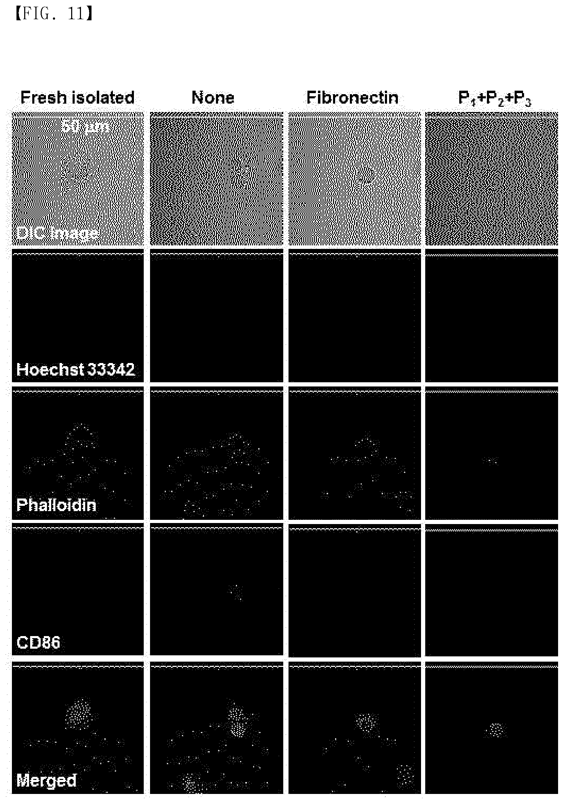

In FIG. 11, culturing spleen dendritic cells (2 × 10⁵/mL) and the prepared peptides were added at a concentration of 200 ug/mL, respectively, and after 6 hours, the expression level of CD86 using a PE-conjugated anti-CD86 antibody (eBioscience) was measured through a confocal microscope after fluorescence staining. After culturing spleen primary dendritic cells for a certain period of time, intracellular CD86, F-actin and nuclear staining was implemented. Expression of actin was stained with FITC-conjugated phalloidin (eBioscience), and nuclear staining was conducted with Hoechst 33342 (Sigma-Aldrich). For this purpose, cells cultured in a 48-well plate were placed in a 4% paraformaldehyde fixing solution, and then fixed at room temperature for 20 minutes.

Then, after washing twice with phosphate buffered saline solution, it was permeabilized with 0.1% Triton X-100 (Sigma-Aldrich) solution at room temperature for 5 minutes and washed twice with phosphate buffered saline solution. In order to remove non-specific staining, after reacting with 0.2% bovine serum albumin (Sigma-Aldrich) solution at room temperature for 20 minutes, it was washed twice with phosphate buffered saline solution. After that, the anti-CD86 antibody and phalloidine were diluted in 0.2% bovine serum albumin solution at a ratio of 1:40 and reacted for 40 minutes, washed 3 times with Tris buffer, placed on a slide glass, followed by counter-staining with Hoechst 33342 solution for nuclear staining of cells. After sealing with a cover glass, the cells were observed using a confocal laser microscope (K1 nanoscope).

### Example 6. TNF-α secretion from cultured spleen dendritic cells and effect of peptides

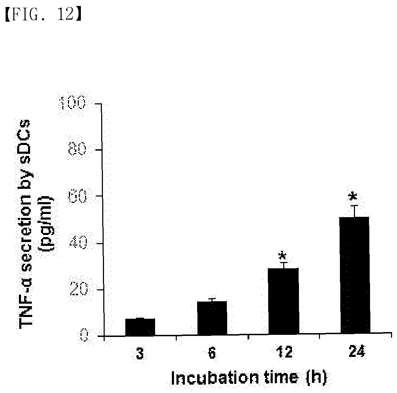

In FIG. 12, spleen primary dendritic cells were dispensed at 1 × 10⁶/mL and cultured for 24 hours, and then a concentration of TNF-α secreted from these cells in the culture medium was measured. As a result, it was confirmed that the secretion increased in a time-dependent manner. The concentration of TNF-α was measured at 450 nm using an ELISA kit (R&D systems) and a microplate analyzer (Synergy H1, Biotek). However, when dendritic cells were dispensed at 2 × 10⁵/mL, CD86 expression was increased but TNF-α secretion was not increased (results not presented). * indicates statistical significance as P <0.05 or less compared with 3 h.

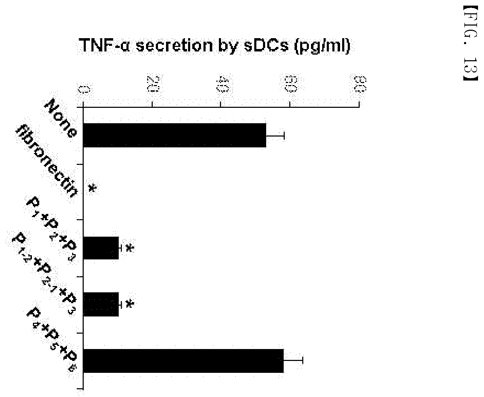

In FIG. 13, spleen primary dendritic cells were dispensed at 1 × 10⁶/mL and cultured for 24 hours. When peptides of P1, P2 and P3 were added or when P1-2, P2-1 and P3 peptides were added, the secretion of TNF-α was significantly decreased. Further, even when fibronectin was added and cultured, TNF-α was hardly measured in the culture medium. Furthermore, when P4, P5 and P6 peptides were added at a concentration of 200 ug/mL, respectively, the secretion of TNF-α was not inhibited. Therefore, although the activation of primary dendritic cells is considered to have no direct relationship with the secretion of TNF-α, an amount of TNF-α secreted from the activated cells is effectively suppressed by fibronectin or a peptide combination. * indicates statistical significance as P <0.05 or less compared with 3 h.

### Example 7. Induction of activation of spleen dendritic cells cultured while adding peptides and fibronectin

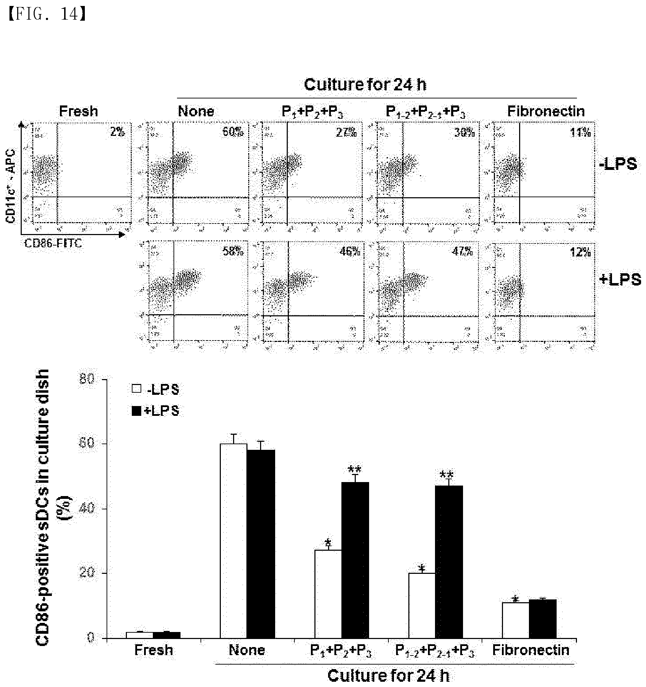

In FIG. 14 , the expression level of CD86 in spleen primary dendritic cells cultured for 24 hours was measured similarly to the expression level of CD86 increased when E. *coli* LPS as an activation-inducing agent was used for treatment at a concentration of 1 µg/mL. Further, it was confirmed that the expression of CD86 was not further increased even when treated by adding LPS under a culture condition for self-activation. CD86 expression of spleen dendritic cells cultured with addition of fibronectin was not increased even when 1 µg/mL LPS was treated. However, in the dendritic cells with suppressed self-activation, in which P1, P2 and P3 peptides were added or P1-2, P2-1 and P3 were added and then cultured, the expression of CD86 was significantly increased by LPS stimulation. * indicates statistical significance as P <0.05 or less compared with None. ** indicates statistical significance as P <0.05 or less compared to -LPS.

### Example 8. Activation level of spleen dendritic cells cultured on nanofiber membranes and effects of peptides and LPS

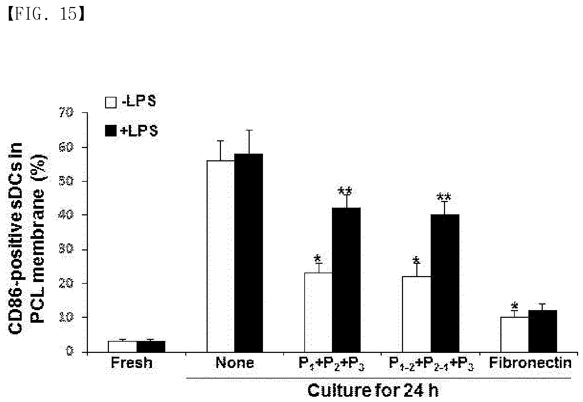

In FIG. 15, even in spleen primary dendritic cells cultured for 24 hours on a nanofiber membrane made of polycaprolactone (PCL), it was found that spontaneous activation occurred like as in the culture conditions of the culture plate, and thus the expression of CD86 was significantly increased. The nanofiber membrane was produced by adding polycaprolactone (Sigma, MW 80,000) to a chloroform solvent at a rate of 15% (w/v), and injecting this solution (5 ml) at a spinning speed of 8 µL/min with a spinning distance of 20 cm using a 25G metal syringe, followed by an electrospinning process using an electrosinning machine (Nano-NC) at 10 KV. The effects of the peptide combination and LPS on CD86 expression of spleen dendritic cells cultured in polycaprolactone nanofibers exhibited the result similar to those obtained in the culture plate shown in FIG. 14. Therefore, the extent of spontaneous activation of the spleen dendritic cells even under three-dimensional culture conditions such as nanofiber membranes was similar to that of two-dimensional culture. * indicates statistical significance as P <0.05 or less compared with None. ** indicates statistical significance as P <0.05 or less compared to -LPS.

### Example 9. Cytokine secretion ability of LPS-stimulated spleen dendritic cells cultured while adding peptides and fibronectin

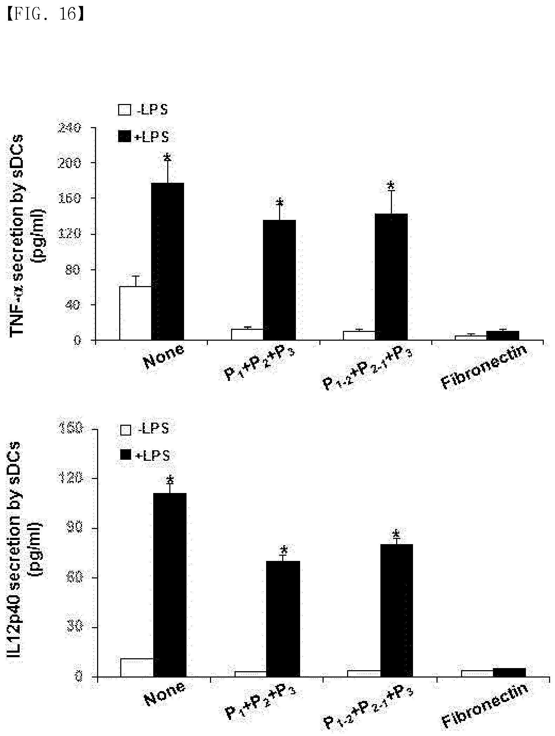

FIG. 16 shows results of measuring whether inflammatory cytokines and Th1-induced cytokines are normally secreted when spleen primary dendritic cells cultured for 24 hours in the state of adding the peptide combination were stimulated with LPS. In the cells cultured with addition of the peptide, the secretion of TNF-α and IL-12p40 was hardly measured while maintaining the inactive form, however, a high concentration was measured in the culture medium by treatment with 1 ug/mL LPS. In comparison, it was found that spleen dendritic cells cultured with addition of fibronectin did not produce these cytokines when stimulated with LPS. Therefore, it could be seen that fibronectin has a disadvantage of inhibiting the activation by stimulants although can maintain the inactivation of spleen dendritic cells. * indicates statistical significance as P <0.05 or less compared to - LPS.

### Example 10. Investigation of effects of peptides and fibronectin on the activation of bone marrow-derived dendritic cells by stimulants

Compared to spleen dendritic cells, when bone marrow-derived DCs (BMDCs) were stimulated with LPS, effects of the peptide combination and the addition of fibronectin were measured.

Mouse bone marrow-derived dendritic cells were obtained by differentiation from mouse bone marrow precursor cells. The femur and tibia of female C57BL/6 mouse were removed, and bone marrow was isolated using 10 mL of EDTA:BSS (Balanced Salt Solution) buffer containing 2% FBS. Red blood cells were removed with erythrocyte lysis buffer (Sigma-Aldrich), washed with EDTA:BSS buffer, followed by filtering cells using a 70 um cell strainer (Falcon). Cells at 3 × 10⁶/mL were dispensed in a 6-well plate, and interleukin-4 (20 ng/mL) (JW Creagene, Korea) and GM-CSF (20 ng/mL) (JW Creagene) were added and cultured for 7 days to induce differentiation. Differentiated dendritic cells were reacted with microbeads bound with CD11c antibody (eBioscience), was separated using CD11c Microbeads Ultrapure (MACS, Milteni Biotec), and then cultured.

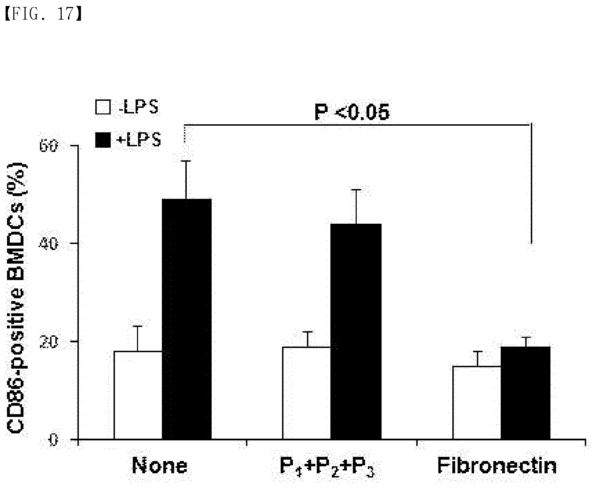

In FIG. 17, while culturing 2 × 10⁵/mL of bone marrow-derived dendritic cells for 24 hours, the peptides of P1, P2 and P3 were each added at a concentration of 200 ug/mL or fibronectin was added at a concentration of 200 ug/mL. When cells were stimulated with LPS (1 ug/mL), CD86 expression was significantly increased by LPS in the case of adding peptides, but CD86 expression did not occur by fibronectin.

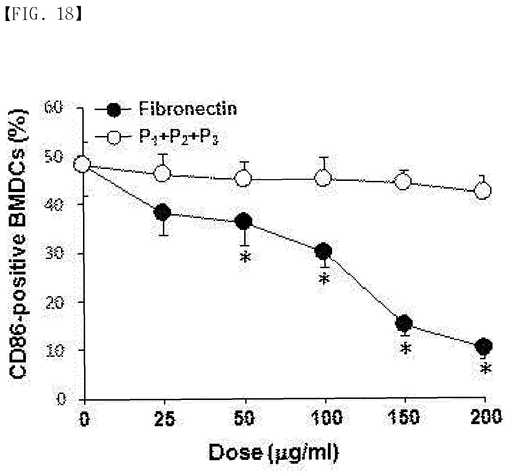

In FIG. 18, when fibronectin was administered by concentration while culturing bone marrow-derived dendritic cells for 24 hours, it could be seen that the increase in the expression of CD86 by LPS (1 gg/mL) was inhibited from a concentration of 50 ug/mL. * indicates statistical significance as P <0.05 or less compared to 0 ug/mL concentration.

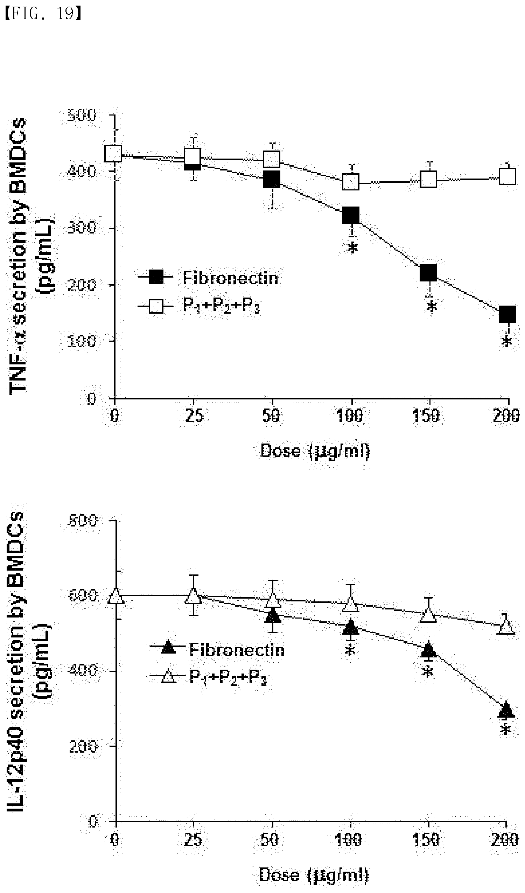

In FIG. 19, when fibronectin was administered by concentration while culturing bone marrow-derived dendritic cells for 24 hours, it could be seen that the secretion of TNF-α and IL-12p40 by LPS (1 ug/mL) was inhibited from a concentration of 100 µg/mL. * indicates statistical significance as P <0.05 or less compared to 0 ug/mL concentration.

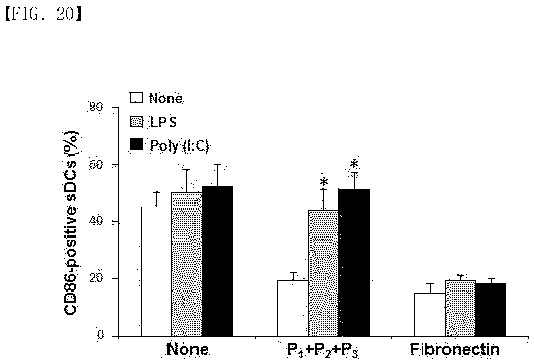

In FIG. 20, compared to LPS binding to Toll-like receptor-4 of immune cells and inflammatory cells including dendritic cells, when poly (I:C) (polyinosinic:polycytidylic acid), which is similar to double-stranded RNA in terms of structure and is known as an immunostimulant that binds to Toll-like receptor-3, was administered at a concentration of 50 ug/mL, the increase in the expression of CD86 in spleen dendritic cells was inhibited when cultured with addition of fibronectin. However, when each of P1, P2 and P3 peptides was added at a concentration of 200 ug/mL, a result of not being affected was obtained. * indicates statistical significance as P <0.05 or less compared with None

### Example 11. T cell proliferation by spleen dendritic cells cultured with peptides and increased cytokine secretion by co-culture

Since the main role of activated dendritic cells is to induce antigen-specific T-cell activation and proliferation according to the stimulatory properties, it was investigated whether self-activated dendrite cells after culturing and dendritic cells activated with LPS after culturing with addition of peptides can induce T cell proliferation by mixed lymphocyte reaction (MLR) test. The spleen dendritic cells isolated from C57B/L6 mice were self-activated for 24 hours, otherwise, when cultured with addition of peptides or fibronectin, the cells were treated with LPS and activated. Spleen T cells from Balb/c mice were isolated using a CD4⁺ /CD8⁺ T cell isolation kit (Miltenyi Biotec, Bergisch-Gladbach, Germany) and MACS column (Miltenyi Biotec), followed by 10 µM CFSE staining (Molecular Probes; Thermo Fisher Scientific Inc., USA). The activated dendritic cells and CFSE-stained T cells were co-cultured at a ratio of 1:20 for 4 days, and the T cell proliferation level was measured as a percentage of cells with decreased CFSE.

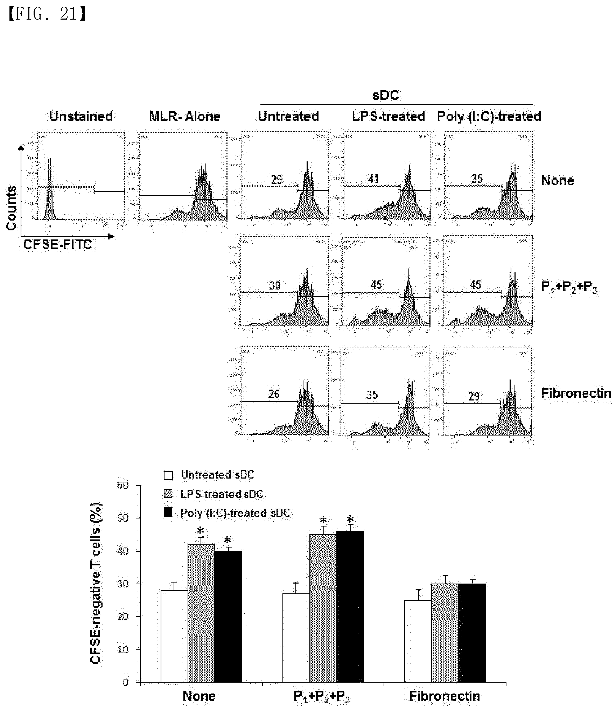

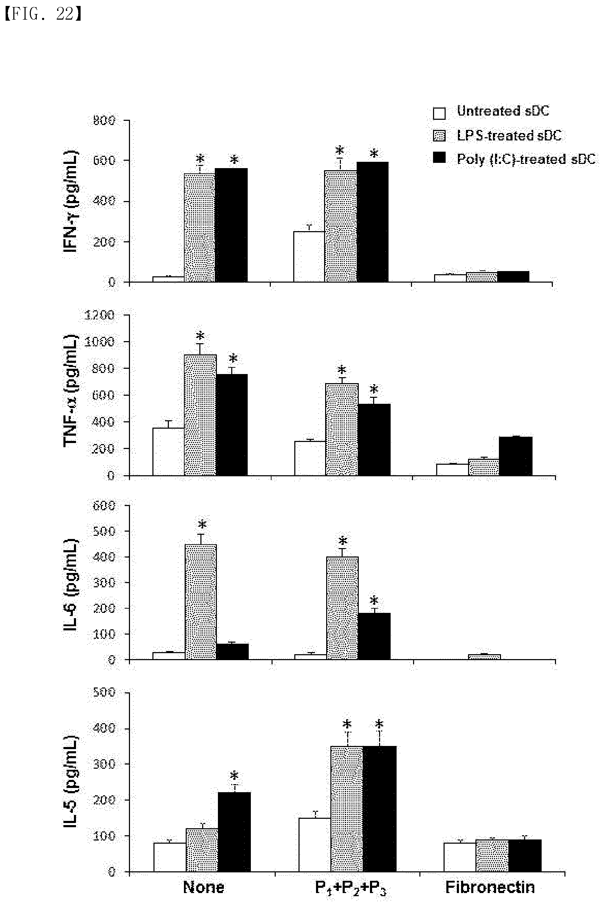

In FIG. 21, dendritic cells activated with LPS or poly (I:C) slightly increased the proliferation of co-cultured spleen T cells compared to spleen dendritic cells of the control not treated with peptide or fibronectin. Further, the spleen dendritic cells activated after treating with peptide also effectively increased T cell proliferation. However, the spleen dendritic cells cultured with addition of fibronectin were not activated by LPS, hence not inducing T cell proliferation. * indicates statistical significance as P <0.05 or less compared with untreated sDC. In FIG. 22, the amount of cytokines secreted after co-culture of spleen dendritic cells and T cells for 4 days was measured by ELISA method. In the case of co-culture of dendritic cells and T cells activated with LPS and poly (I:C) while culturing with the addition of control or peptide, high concentrations of interferon-gamma (IFN-γ), TNF-α, IL-6 and IL-5 were secreted. However, the secretion of these cytokines was extremely low in the co-culture of activated spleen dendritic cells and T cells with addition of fibronectin. * indicates statistical significance as P <0.05 or less compared to untreated sDC.

### Example 12. Increased viability of spleen dendritic cells cultured with peptides

Dendritic cells are characterized by rapid cell death during culture while self-activation occurs. In addition to the inhibition of self-activation, it was measured whether the viability of dendritic cells is changed. Cell viability was determined by staining of culturing cells with 7-AAD and measuring the number of cells not stained with the above fluorescent substance.

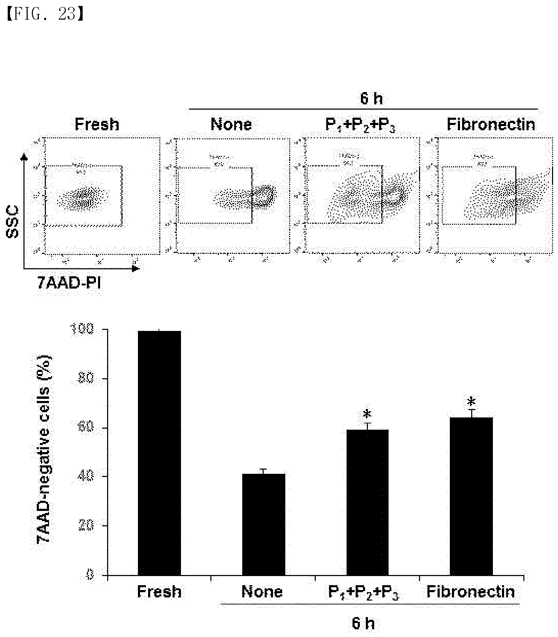

In FIG. 23, the spleen dendritic cells significantly decreased cell viability after 6 hours of culture, but the number of 7-AAD-negative surviving cells was significantly increased when cultured with addition of peptides of P1, P2 and P3 or fibronectin. * indicates statistical significance as P <0.05 or less compared to untreated sDC.

### Example 13. Measurement of effect of linker peptide composed of three peptides on CD86 expression in cultured primary dendritic cells

As a linker, an amino acid linker GS (Gly-Ser) and a flexible linker GGGSGGGS were used to link three types of peptides, RGD (P₁), LDV (P₂), and PHSRN (P₃), thereby producing PHSRNGSRGDGSLDV (named as LP₁, SEQ ID NO: 12), PHSRN GGGSRGDGGGSLDV (named as LP₂, SEQ ID NO: 13), PHSRNGGGSGGGS RGDGGGSLDV (named as LP₃, SEQ ID NO: 14). The production of the linker peptide was requested to Peptron Co., Ltd. (Daejeon, Korea), and the product was purchased from the same. This product was manufactured as SHIMADZU LCMS-2020. As controls, S-P₁ (SEQ ID NO: 15), S-P₂ (SEQ ID NO: 16), and S-P₃ (SEQ ID NO: 17) as scrambled peptides in which the sequences of amino acids constituting the P₁, P₂ and P₃ peptides are mixed, and linker peptides composed of the scrambled peptides S-LP₁ (SEQ ID NO: 21), S-LP₂ (SEQ ID NO: 22), and S**-**LP₃ (SEQ ID NO: 23) were produced and used. The scrambled peptides or scrambled linker peptides have the same number of amino acids, molecular weight, isoelectric point and hydrophilicity as the original peptides. The sequences of these peptides are shown in Table 1 below.

**[TABLE 1]**

| **Peptide** | **Amino Acid Sequence** | **position in Fibronectin** | **M. W. (g/mole)** | **Molecular Formula** | **Isoelectric Point (pI)** | **Average Hydrophilicity** | |
|---|---|---|---|---|---|---|---|
| P₁ | RGD | 1615-1617 | 346 | C₁₂H₂₂N₆O₆ | 6.8 | 2.0 | SEQ ID NO: 1 |
| P₂ | LDV | 2102-2104 | 346 | C₁₅H₂₇N₃O₆ | 3.1 | -0.1 | SEQ ID NO: 2 |
| P₃ | PHSRN | 1498-1502 | 609 | C₂₄H₃₉N₁₁O₈ | 11.1 | 0.6 | SEQ ID NO: 3 |
| LP₁ | PHSRNGSRGDGSLDV | n/a | 1553 | C₆₁H₁₀₀N₂₄O₂₄ | 7.8 | 0.6 | SEQ ID NO: 12 |
| LP₂ | PHSRNGGGSRGDGGGSLDV | n/a | 1781 | C₆₉H₁₁₂N₂₈O₂₈ | 7.8 | 0.5 | SEQ ID NO: 13 |
| LP₃ | PHSRNGGGSGGGSRGDGGGSLDV | n/a | 2039 | C₇₈H₁₂₆N₃₂O₃₃ | 7.8 | 0.4 | SEQ ID NO: 14 |
| S-P₁ | GDR | n/a | 346 | C₁₂H₂₂N₆O₆ | 6.8 | 2.0 | SEQ ID NO: 15 |
| S-P₂ | VLD | n/a | 346 | C₁₅H₂₇N₃O₆ | 3.1 | -0.1 | SEQ ID NO: 16 |
| S-P₃ | SNPRH | n/a | 609 | C₂₄H₃₉N₁₁O₈ | 11.1 | 0.6 | SEQ ID NO: 17 |
| S-LP₁ | NRPHSGSDRGGSVDL | n/a | 1553 | C₆₁H₁₀₀N₂₄O₂₄ | 7.8 | 0.6 | SEQ ID NO: 21 |
| S-LP₂ | NRPHSGGGSDRGGGGSVDL | n/a | 1781 | C₆₉H₁₁₂N₂₈O₂₈ | 7.8 | 0.5 | SEQ ID NO: 22 |
| S-LP₃ | NRPHSGGGSGGGSDRGGGGSVDL | n/a | 2039 | C₇₈H₁₂₆N₃₂O₃₃ | 7.8 | 0.4 | SEQ ID NO: 23 |

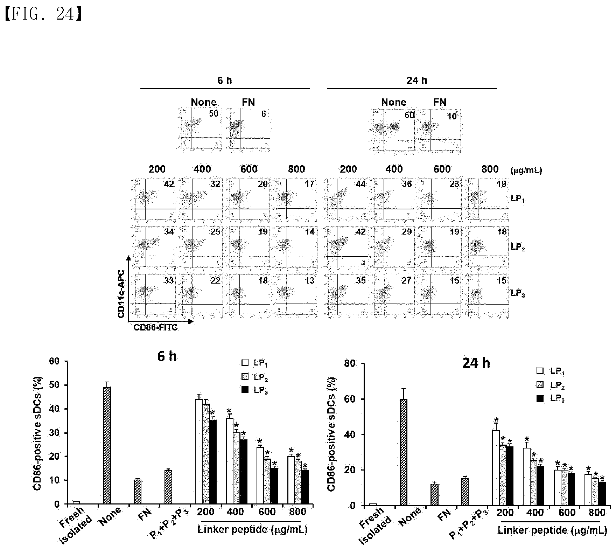

In FIG. 24, different concentrations of LP₁, LP₂ and LP₃ were added to reach a concentration of 800 ug/mL, and spleen dendritic cells (2 × 10⁵/mL) were cultured for 6 hours or 24 hours, and the CD86 expression level was measured by flow cytometry (MACSQuant VYB flow cytometer, Miltenyi Biotec). The measured results were analyzed using FlowJo v10 (Tree Star, Ashland). The increase in CD86 expression, which is increased by self-activation after culture, was inhibited by LP₁, LP₂ and LP₃ in a concentration-dependent manner, and LP₃ showed the greatest self-activation inhibitory effect. * has a statistical significance as P <0.05 or less compared to None.

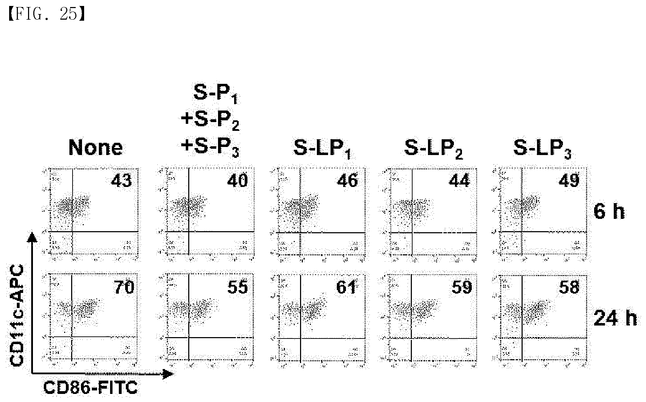

In FIG. 25, when spleen primary dendritic cells were dispensed at 2 × 10⁵/mL and cultured for 24 hours while scrambled peptides of S-LP₁, S-LP₂ and S-LP₃ were each added at a concentration of 800 ug/mL, the increase in the expression of CD86 was not inhibited. Further, the expression of CD86 was not reduced even when the scrambled peptides S-P₁, S-P₂ and S-P₃ were simultaneously treated at a concentration of 600 ug/mL, respectively.

### Example 14. Measurement of fluorescence staining for CD86 expression in spleen dendritic cells cultured with the addition of linker peptide

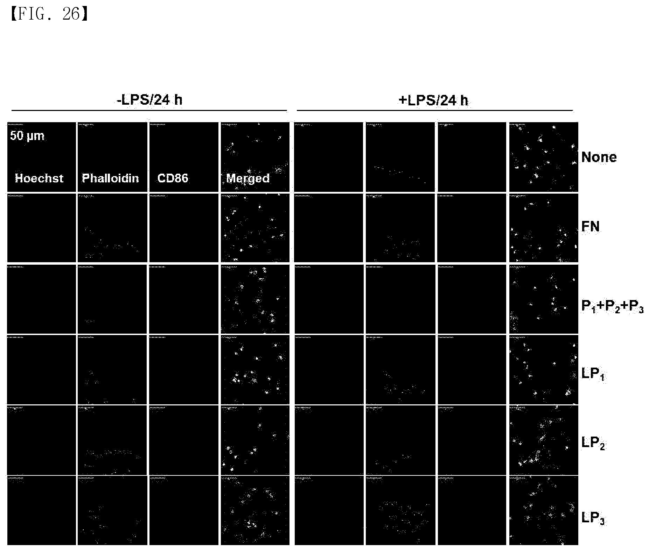

In FIG. 26, spleen dendritic cells (2 × 10⁵/mL) were dispensed in a 48-well plate, and linker peptides were added at a concentration of 400 ug/mL, followed by culturing for 24 hours. Activation of spleen dendritic cells was induced by adding 1 ug/mL of LPS to the culture medium. The cultured cells were placed in a 4% paraformaldehyde fixing solution and fixed at room temperature for 20 minutes. After fluorescence staining using PE-conjugated anti-CD86 antibody (eBioscience), it was measured with a confocal microscope (K1 Nanoscope, Korea). Intracellular actin expression was stained with FITC-conjugated phalloidin (eBioscience), and nuclear staining was conducted with Hoechst 33342 (Sigma-Aldrich). CD86 antibody and phalloidin were diluted 1:40 in 0.2% bovine serum albumin solution and used. When only spleen dendritic cells were cultured in the culture medium, CD86 expression was significantly increased. However, in both of the cases where fibronectin or three types of peptides were added and where the linker peptides LP₁, when LP₂ and LP₃ were added, CD86-expressing cells were almost not observed. Further, when LPS was added, CD86-expressing cells were almost not measured if fibronectin is contained. However, in a state of culturing with adding a combination of three types of peptides (P₁ + P₂ + P₃) or linker peptides, the number of CD86-expressing cells was significantly increased by LPS treatment.

### Example 15. Effect of linker peptide on TNF-α secretion by cultured spleen dendritic cells

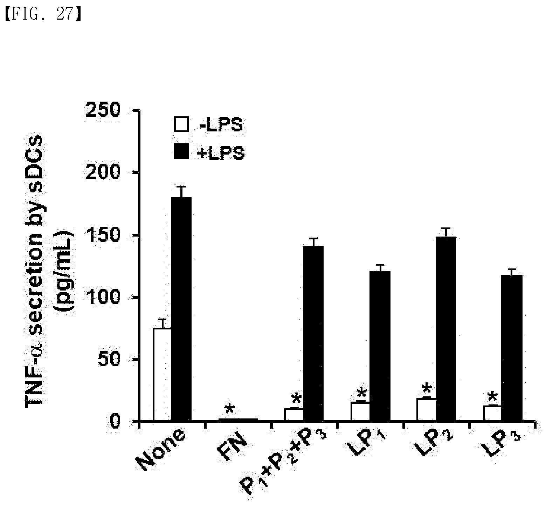

In FIG. 27, an ELISA kit (R&D systems) was used to measure the concentration of TNF-α secreted into the culture medium when spleen primary dendritic cells were dispensed at 2 × 10⁵/mL and cultured for 24 hours. The concentration was determined at 450 nm by a microplate analyzer (Synergy H1, Biotek). When only culturing the dendritic cells, the concentration of TNF-α was measured to be 75 ± 10 pg/mL and was significantly increased by LPS stimulation. When fibronectin, a combination of three types of peptides (P₁ + P₂ + P₃) and linker peptides LP₁, LP₂ and LP₃ were added, it was found that TNF-α secretion hardly occurred. In the case of being subjected to LPS treatment, like as the combination of three types of peptides (P₁ + P₂ + P₃), even when linker peptides were added, the secretion of TNF-α was significantly increased compared to addition of fibronectin. * has a statistical significance as P <0.05 or less compared to None.

### Example 16. Measurement of T cell activation by spleen dendritic cells cultured with linker peptide

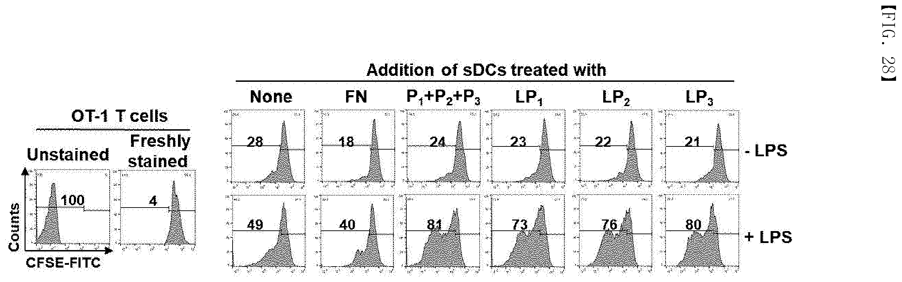

In FIG. 28, it was investigated whether spleen dendritic cells, in which self-activation does not occur by the linker peptide during culture, and dendritic cells activating with LPS, can induce T cell proliferation. The spleen dendritic cells (2 × 10⁶/mL) were self-activated in a 96-well plate for 24 hours, or self-activation was inhibited by adding a linker peptide, and then either not treated with LPS or stimulated with LPS for 24 hours. Dendritic cells were cultured with 10 µM ovalbumin (OVA) peptide SIINFEKL (InvivoGen, San Diego, California, USA) for 1 hour. CD8⁺ T cells were isolated from OT-I transgenic mice using a CD8 T cell isolation kit (Miltenyi Biotec) and fluorescence-stained with 10 µM carboxyfluorescein succinimidyl ester (CFSE) for 10 minutes. After co-culture of CD8⁺ T cells (1 × 10⁵) and DCs (5 × 10³) treated with OVA peptide at a ratio of 1:20 for 4 days, a proliferation level of T cells was measured in percentage of cells that are negative for CFSE fluorescence-staining.

Dendritic cells that had undergone self-activation did not induce T cell proliferation if LPS treatment is not performed. When stimulated with LPS, self-activated cells or dendritic cells cultured with fibronectin had very weak ability to induce T cell proliferation, whereas LPS-stimulated dendritic cells cultured with a combination of the three peptides or linker peptides significantly increased T cell proliferation.

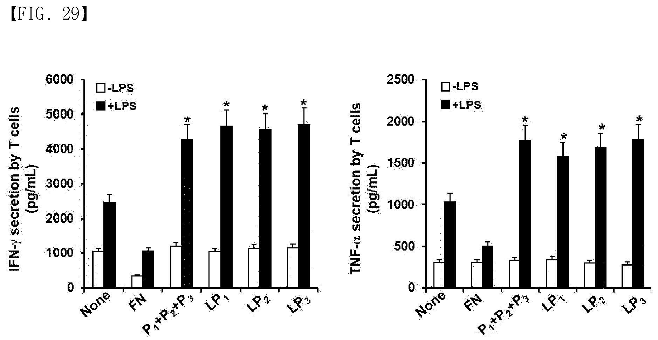

In FIG. 29, the concentrations of interferon-gamma (IFN-γ) and TNF-α secreted into the culture medium were measured by ELISA after co-culture of spleen dendritic cells and T cells for 4 days under the same conditions as for T cell proliferation measurement. Self-activated dendritic cells secreted 2.5 times more interferon-gamma and TNF-α when co-cultured with T cells after stimulation with LPS. On the other hand, dendritic cells cultured with adding a combination of three types of peptides or cultured with LP₁, LP₂, LP₃ linker peptides showed increase in the secretion of these cytokines by 4.5 times or more when stimulated with LPS and then co-cultured with T cells. * is less than P <0.05 compared to None, which is statistically significant.

## Claims

1. A composition for inhibiting self-activation of dendritic cells, comprising a fibronectin-derived peptide which binds to an integrin binding site between the dendritic cells.

2. The composition according to claim 1, wherein the peptide is a peptide consisting of an amino acid sequence including any one of SEQ ID NOs: 1 to 3.

3. The composition according to claim 1, wherein the peptide has any one amino acid sequence of SEQ ID NOs: 1 to 3, and has a length of 3 to 11aa.

4. The composition according to claim 1, wherein the peptide is at least one selected from the group consisting of any one amino acid sequence of SEQ ID NOs: 1 to 7.

5. The composition according to claim 1, wherein the peptide is a peptide in which two or more amino acid sequences comprising any one of SEQ ID NOs: 1 to 3 are linked by a linker.

6. The composition according to claim 5, wherein the linker consists of amino acids G and S, and has a length of 2 to 8aa.

7. The composition according to claim 5, wherein the linker consists of an amino acid sequence including any one of SEQ ID NOs: 8 to 11.

8. The composition according to claim 5, wherein the peptide has any one amino acid sequence of SEQ ID NOs: 12 to 14.

9. The composition according to claim 1, wherein the peptide is contained in a concentration of 200 ug/mL or more based on 2 × 10 ⁵/mL of the dendritic cells to be treated.

10. The composition according to claim 1, wherein the dendritic cells are derived from monocytes, spleen or lymph nodes.

11. The composition according to claim 1, wherein the composition is for use *in vitro.*

12. A method of culturing dendritic cells, comprising treating the isolated dendritic cells with the composition according to any one of claims 1 to 11.

13. The method according to claim 12, further comprising inducing activation of the dendritic cells by treating the dendritic cells, which were treated with the composition, with an immunostimulant.

14. The method according to claim 13, wherein the immunostimulant is at least one selected from the group consisting of a bacterial agent, CD40 ligand, TNF-α, IL-1β, LPS and poly (I:C).

15. A method of activating immune cells, comprising:
treating the isolated dendritic cells with the composition according to any one of claims 1 to 11 and culturing the dendritic cells in an inactivated state;
activating the dendritic cells; and
co-culturing the activated dendritic cells and immune cells.

16. A dendritic cell composition, comprising:
a fibronectin-derived peptide which binds to an integrin binding site between the dendritic cells according to any one of claims 1 to 11; and dendritic cells mixed therewith.

17. A pharmaceutical composition for treating or preventing cancer, infectious disease, chronic inflammatory disease, or autoimmune disease, comprising the cell composition according to claim 16 as an active ingredient.
